# EUROPEAN PATENT APPLICATION

(11) **EP 4 817 619 A1**
(43) Date of publication of application: **07.10.2026**
(21) Application number: 25167552.6
(22) Date of filing: 31.03.2025
(51) Int. Cl.: A61K 33/22, A61K 45/06, A61P 27/02, A61K 8/19, A61Q 1/10

(54) **COMPOSITION FOR KERATOPIGMENTATION WITHOUT TITANIUM DIOXIDE**

(71) Applicant: Laboratoires BIOTIC Phocea, 13008 Marseille (FR)
(72) Inventor: TIZIANO, Sandie, 13008 Marseille (FR)
(74) Representative: Barbot, Willy

(57) **Abstract**

The present invention concerns a new composition for keratopigmentation, whose pigments comprise boron nitride particles instead of titanium dioxide particles.

## Description

### Technical field

The present invention relates to the field of keratopigmentation.

### Background

Keratopigmentation (KTP), better known as corneal tattooing, has been used initially for cosmetic management of corneal opacities.

Today, various KTP techniques exist and are disclosed in the recent review of D'ORIA et al. (Ophthalmol. Ther., vol.11(2), p: 465-477, 2022). Usually, dyes are applied to the anterior stroma following epithelial debridement or trans-epithelial needle puncture (superficial technique).

Among these technics, Femtosecond laser-assisted keratopigmentation (FLAK) has been recently introduced for performing KTP to restore cosmetic and functional problems, by providing uniform lamellar depth and a smoother stromal bed. As an example, patent application US 2014/107,631 discloses a FLAK method creating a stromal tunnel, wherein pigments are injected and delivered to the superficial layers of the cornea.

The high safety and efficacy of FLAK make its elective application the best option for a suitably selected patient who wishes to change the apparent color of the eye.

Nevertheless, some complications still occurred after the procedure, notably some color fading.

Accordingly, there is a need for new developments limiting such complications.

### Summary of the invention

The inventors have now demonstrated that boron nitride particles are safer than titanium dioxide particles and provide a better adherence and color time stability in keratopigmentation to the pigments 'mixtures incorporating said particles instead of titanium dioxide particles.

Accordingly, a first object of the invention relates to a composition for keratopigmentation comprising pigments, characterized in that said pigments comprise boron nitride particles.

Advantageously, said pigments do not comprise any titanium dioxide particles.

A second object of the invention relates to the corresponding composition for pharmaceutical use (i.e., as a medicament).

A third object of the invention relates to a composition as disclosed previously for treating an iris defect in a subject by keratopigmentation.

A fourth object of the invention relates to a method of keratopigmentation comprising the step of injecting an effective amount of the abovementioned composition to a subject for obtaining a desired iris coloration.

### Detailed description

As used herein, "pigments" refer to natural or synthetic insoluble colorants that are used to impart visual properties on a wide range of products. Pigments are completely or nearly insoluble and chemically unreactive in water or another medium. In contrast, dyes are colored substances which are soluble or go into solution at some stage in their use. Dyes are often organic compounds whereas pigments are often inorganic.

Pigments that can be used in the composition of the invention are available in powder form and are well known from the skilled person in tattoo.

Preferably, the pigments are inorganic and can be easily identified by the skilled person. Such inorganic pigments typically have CI numbers in the 77000 to 77999 range. As an example, such pigments are selected in the group comprising Black 6/7 (CI 77266), Black 11 (CI 77499), Blue 29 (CI 77007), Green 17 (CI 77288), Green 18 (CI 77289), Manganese violet (CI 77742), Red 101 (CI 77491), violet 27 (CI 77510), Yellow 42 (CI 77492), Yellow 119 (CI 77496), and any combination thereof.

Advantageously, the pigments represent from 1.0 to 30% (w/w) of the composition, preferably from 1.5 to 25%, typically from 2.0 to 20%, and most preferably from 2.5 to 15% (w/w) of the composition.

To deliver the pigments to the desired substrate (cornea), the pigment must be transported in a liquid form. Now, the pigment particles may form agglomerates during their manufacturing process. Accordingly, such pigment agglomerates must be broken down during "deagglomeration" fur using them in the composition of the invention, mostly by inserting mechanical forces (impact and shear forces).

Advantageously, the pigments in the composition are in the form of particles, whose size is comprised between 50 nm and 20 µm, preferably between 100 nm and 5 µm, and most preferably between 150 nm and 2 µm.

Still advantageously, the particle size distribution D90 (90 vol. %) of the pigments is less than 5 µm, preferably less than 2 µm, and most preferably than 1 µm.

As used herein, D90 refers to the particle size range at which 90% of the particles are smaller than. Such particle size distributions may be measured using any device and method known for this use in the art. For example, particle size may be measured by Laser Diffraction.

Concerning the new tattoo pigment boron nitride, it is a compound of boron and nitrogen with the chemical formula BN. As disclosed herein, boron nitride (and boron nitride particles) is considered as a pigment.

Because of its high stability, BN is essentially used as a lubricant at both low and high temperatures (up to 900 °C, even in an oxidizing atmosphere). Now, it is also used in cosmetics since the 1990s as an absorbent, an opacifier or as a skin conditioning agent.

Boron nitride exists in various crystalline forms that are isoelectronic to a similarly structured carbon lattice. Such various boron nitride crystalline forms include hexagonal boron nitride, cubic boron nitride and wurtzite boron nitride.

The hexagonal boron nitride presents a hexagonal structure analogous to the one of graphite. This crystalline form of boron nitride is the most stable and abundant. The hexagonal boron nitride is generally called hBN. The hexagonal boron nitride is the softest among BN crystalline forms and is therefore used as a lubricant and an additive to cosmetic products.

The cubic boron nitride presents a cubic structure analogous to the one of diamond. The cubic boron nitride is generally called cBN. This cubic crystalline form of boron nitride is softer than diamond, but its thermal and chemical stability is superior.

The wurtzite boron nitride presents a wurtzite structure analogous to the one of lonsdaleite. This crystalline form of boron nitride is the rarest and is generally called wBN. This cubic crystalline form of boron nitride is slightly softer than cubic crystalline boron nitride.

Preferably, boron nitride (BN) refers to hexagonal boron nitride (hBN).

Boron nitride is available in particulate form consisting of high-quality crystalline flakes of boron nitride with outstanding purity. Such available particles are defined under Standard Platelets grades (SP), with no agglomeration and a tight particle size distribution around their average size. Such Boron nitride Standard Platelets grades are disclosed in the following table.

| Grade | | Typical D10 (µm) | D50 (µm) | Typical D90 (µm) |
|---|---|---|---|---|
| SP1 | | 0.5 | 1.0 | 2.0 |
| SP2 | | 1.0 | 2.0 | 4.0 |
| SP5 | | 3.0 | 5.0 | 10 |
| SP6 | | 4.0 | 6.0 | 12 |
| SP8 | | 4.5 | 8.0 | 16 |
| SP12 | | 6.0 | 12 | 20 |
| SP16 | | 8 | 16 | 30 |
| SP30 | | 15 | 30 | 50 |

D10: This indicates that 10% of the particles have a particle size smaller than this value. D50: Also known as "average size," this means that 50% of the particles are smaller than this value. D50 reflects the average size of the entire particle population. D90: This indicates that 90% of the particles are smaller than this value. D90 primarily reflects the proportion of larger particles in the population and can be used to assess the roughness of the population.

Preferably, boron nitride particles are selected in the group comprising SP1, SP2, SP5, SP6 or SP8 boron nitride, and most preferably boron nitride particles are SP2 boron nitride.

Advantageously, boron nitride particles represent from 0.1 to 20% (w/w) of the composition, preferably from 0.5 to 15, and most preferably from 1.0 to 10% (w/w) of the composition.

Titanium dioxide is a white solid that is insoluble in water with the chemical formula TiO2. As a pigment, titanium dioxide is called titanium white, Pigment White 6 (PW6) or CI 77891. This pigment is the most widely used white pigment because of its brightness and of its opacifier capacity. It has been estimated that titanium dioxide is used in two-thirds of all pigments and in a large products scope such as sunscreens, paints, coatings, plastics, papers, inks, foods, supplements, and medicines.

Nevertheless, the European Food Safety Authority (EFSA) ruled that as a consequence of new understandings of nanoparticles, titanium dioxide particles could "no longer be considered safe " since genotoxicity-which could lead to carcinogenic effects-could not be ruled out.

It is presently demonstrated that the titanium dioxide particles can be safely replaced by boron nitride particles inducing less toxicity. Furthermore, such replacement corresponds to better pigments adherence in keratopigmentation with increased non-fading characteristics of said pigments.

Thus, and according to a first preferred embodiment, the composition of the invention is devoided of titanium dioxide particles (CI 77891).

As used herein, "composition devoided of titanium dioxide particles" refers to a composition comprising less than of 0.1% (w/w), preferably less than 0.01% (w/w), and most preferably less than 1 ppm (w/w).

The composition of the invention comprises a pigments' carrier.

Pigments' carriers that can be used for keratopigmentation can be simply identified by the killed person. As an example of such carrier, on can cite water, glycerin, propane-1,2-diol, Propane-1,3-diol, and combination thereof.

Advantageously, the carrier represents from 60 to 99% (w/w) of the composition, preferably from 80 to 98% (w/w), and most preferably from 85 to 97% (w/w) of the composition.

The composition of the invention may further comprise one or more additive such as dispersants, humectants, or pH regulators.

A dispersant, also referred to as a stabilizer, may be any polymer known in the art that stabilizes the dispersed pigment particles either by electrostatic and/or steric interactions. Examples of such dispersants include polyethylene glycol (PEG) polymers and polyvinylpyrrolidone (PVP) polymers.

A humectant may prevent drying of the cornea and improve redispersibility of pigments. Examples of such dispersants include hyaluronic acid and sorbitol.

A pH regulator may prevent the pH of the composition to damage the cornea. Example of such pH regulators include acetic acid, ammonium carbonate, ammonium phosphate, calcium acetate, calcium carbonate, citric acid, lactic acid and sodium hydroxide.

Advantageously, said additives represents less than 15% (w/w) of the composition, preferably less than 10% (w/w), and most preferably less than 5% (w/w) of the composition.

The composition of the invention herein has reduced impurity content by the use of carefully developed processing procedures. Raw material vendors should adhere to consistent quality management systems and practices to ensure constancy in the product (e.g., guidelines from ISO 9001). ISO is the International Organization for Standardization that provides specific requirements covering quality and safety aspects of all raw materials as well as finished goods. The composition of the invention may meet the purity requirements set out by the COMMISSION REGULATION (EU) 2020/2081 ("EU Regulation"). According to the EU Regulation, pigment dispersions must not contain the following impurities above the stated limits:
1. Free primary aromatic amines (PAA) according to Annex XVII to Regulation (EC) No. 1907/2006 (Reach): limit 5 ppm per PAA
2. Heavy metals according to appendix 13 of Annex XVII to Regulation (EC) No. 1907/2006: limit for each heavy metal species is specified in referenced regulation.
3. Polycyclic aromatic hydrocarbons (PAH): limit 0.5 ppm per PAH compound, 5 ppb for Benzo[a]pyrene.
4. Methanol: limit 11% by weight
5. Dyes, pigments, and colorants listed in Appendix 13 of Annex XVII to Regulation (EC) No. 1907/2006: Limit 0.1%
6. Preservatives, phthalates, and other substances with the following hazardous classification according to Part 3 of Annex VI to Regulation (EC) No 1272/2008:
6a. Carcinogen category 1A, 1B, or 2, or germ cell mutagen category 1A, 1B or 2: limit 0.00005% by weight.
6b. Reproductive toxicant category 1A, 1B or 2: limit 0.001% by weight.
6c. Skin sensitizer category 1, 1A or 1B: limit 0.001% by weight.
6d. Skin corrosive category 1, 1A, 1B or 1C, or skin irritant category 2, or as serious eye damage category 1 or eye irritant category 2: limit 0.1% by weight if used solely as a pH regulator, limit 0.01% by weight in all other cases.
7. Substances listed in Annex II to Regulation (EC) No 1223/2009 of the European Parliament and of the Council with a limit of 0.0005% by weight.
8. Substances listed in Annex IV to Regulation (EC) No 1223/2009 for which any of the following conditions is specified in at least one of the columns, g, h, and I of the table in that Annex:
8a. "Rinse-off products": limit 0.00005% by weight.
8b. "Not to be used in products applied on mucous membranes": limit 0.00005% by weight.
8c. "Not to be used in eye products"; limit 0.00005% by weight.

The composition is formulated so as present a viscosity comprised between 50 mPa.s to 5,000 mPa.s, preferably between 100 mPa.s to 2,500 mPa.s, and most preferably between 200 mPa.s to 2,000 mPa.s as measured using any standard viscometer, which may also be referred to as a viscosity meter or rheometer.

The composition of the invention may best be manufactured in a controlled environment. Manufacturing facilities may be certified under ISO 22716:2007 for Cosmetic Products, and under ISO 13485:2016 for medical devices, or any similar or updated versions of these standards.

The method of making the composition may include mixing the pigments with the other ingredients using, for example a high shear mixer. The mixture temperature may be maintained at ambient room temperature (about 20° C. to about 25° C.), or it may be warmed up to 43° C. The mixing may continue until the composition becomes homogenous. Homogeneity may be checked by qualitative method of ink spreading onto a flat surface and observing any aggregates.

The composition should be void of any microbiological contamination, including the microbiological classes listed below. Limit of detection should be <10 colony forming units/gram (CFU/g).

Microbiological Classes to be Tested:
1. Spores of aerobes spore-forming
2. Spores of anaerobes spore-forming
3. Bacillus cereus presumptive
4. Sulphite reducing clostridia
5. Total viable count, aerobes mesophil 30° C.
6. Total viable count, anaerobes mesophil 30° C.
7. Pseudomonas sp.

The composition for keratopigmentation must be sterilized prior to retail distribution.

Advantageously, the composition is a sterilized composition.

The composition of the invention is very stable following a sterilization by irradiation.

Still advantageously, the sterilization of the composition is accomplished by X-ray irradiation or gamma irradiation.

Three main surgical techniques have been described for keratopigmentation, which can be divided between intrastromal and superficial keratopigmentation.

The recommended and most commonly used in cases of cosmetic keratopigmentation is femtosecond laser-assisted intrastromal keratopigmentation (FLAK), in which a circular stromal tunnel is created with a femtosecond laser, with pupil diameter set to an inner diameter of 5.5 mm and an outer diameter of 9.5 mm: the tunnel is eventually opened to the periphery of the cornea with a lamellar dissector until reaching the limbus, followed by the injection of the pigment through the superior incision.

Manual intralamellar keratopigmentation (MIK) is a surgical alternative, wherein two to four freehand incisions are performed from the limbus to the border of a previously marked pupil. The cornea is then dissected intralamellarly and circumferentially using a microcrescent blade and helicoidal intrastromal corneal dissectors. As previously, the pigment is injected through the superior incision.

Finally, superficial automated keratopigmentation (SAK) (ALIO et al., Br. J. Ophthalmol., vol.94, p: 245-249, 2010), wherein the pigment is applied with a micropunctural device (VISSUM EYE MP SYSTEMS) in the cornea of the eye. This method adapts the conventional tattooing technique to the performance of a corneal tattoo and therefore has clearly artisanal and artistic aspects. The center of the cornea is previously marked with a caliper, and the pupil size is determined by an optic zone marker (KATENA). SAK is recommended in cases of deep and dense corneal opacities or for fine-tuning the details of the iris pattern, but not as the first treatment choice for a patient seeking an elective change of eye color.

Preferably, the keratopigmentation is femtosecond laser-assisted intrastromal keratopigmentation (FLAK). Patent application US 2014/0107631 provides FLAK protocols examples.

As used herein, a subject refers to a mammal, preferably to a human.

Now, said subject may require such keratopigmentation only for esthetic purposes (i.e., obtaining a desired iris coloration). Accordingly, the method of keratopigmentation is cosmetic keratopigmentation.

Alternatively, the subject may suffer from an iris defect.

As used herein, an iris defect may relate to corneal opacities (e.g., resulting from leukoma), corneal scars, aniridias, colobomas, heterochromias, and iris lacks pigmentation (e.g., Albino subjects).

The composition of the present invention is administered in the cornea by keratopigmentation in an "effective amount" for performing the desired iris coloration. An effective amount is the quantity of composition necessary to obtain the desired iris coloration in a subject. Amounts effective for keratopigmentation will, of course, depend on the subject, on the desired coloration, as well as on used keratopigmentation method.

In a relation to a human subject, an effective amount of composition for administration corresponds to 0.2 to 2.0 mL per eye, preferably to 0.5 to 1.0 mL.

In the following, the invention is described in more detail. Yet, no limitation of the invention is intended by the details of the examples. Rather, the invention pertains to any embodiment which comprises details which are not explicitly mentioned in the examples herein, but which the skilled person finds without undue effort.

### Examples

### 1. Cytotoxicity

BALB/C 3T3 clone A31 cells were seeded at an initial density of 30,000 cells/cm². The cultures are incubated at 37°C in a humid atmosphere containing 5% (v/v) CO2 for 24 hours. The medium is removed and then replaced with 2 mL of a volume-to-volume mixture, maintained at 37°C, of 2-fold concentrated DMEM (supplemented with 20% FCS (v/v) and 2% antibiotics (v/v)), and 2% agar (w/v) in ultrapure water previously autoclaved for 20 minutes at 120°C.

Compositions of 1,3-propanediol comprising 34.3% (w/w) of SP2 boron nitride or 38.4% (w/w) of titanium dioxide particles (CI 77891) were prepared. Then, one sterile inert filter (1 cm2 disc, 1.2 cm in diameter) was impregnated with complete culture medium (blank) or with a dilution (1/20 or 1/30 in complete culture medium) of the composition of titanium dioxide particles or of SP2 boron nitride.

After waiting for about ten minutes under the laminar flow hood to allow the agarose gel to solidify at room temperature, the test solutions (diluted 1/20 and 1/30 then placed on the disc), the blank (disc impregnated with complete culture medium), the absolute negative control (well without disc) and the positive control (1 cm2 latex disc) are incubated (n=3 for all conditions) in a humid atmosphere containing 5% (v/v) of CO2 for 24 hours.

At the end of the incubation period, the samples are carefully collected after identifying their outlines with a permanent marker.

2 mL of a 0.01% (w/v) Neutral Red solution in the culture medium is added, followed by incubation for 2 hours at 37°C in the dark. The dye solution is then removed, and the wells are examined under a microscope. After staining the cells, an unstained area (lysis zone) may appear in the center of the well. The largest and smallest diameters of this area are measured, and the mean diameter (MD) is calculated. The result is the arithmetic mean of the mean diameters measured across the three wells. This value is expressed in centimeters. The cytotoxicity of the test substance is given by the following scale.

| Mean diameter (MD) of the lysis zone (cm) | Classification |
|---|---|
| Not detectable | no cytotoxicity |
| Just under the disk | mild cytotoxicity |
| MD ≤ 1.2 | low cytotoxicity |
| 1.2 ≤ MD ≤ 3.2 | moderate cytotoxicity |
| MD ≥ 3.2 | strong cytotoxicity |

The results have shown that each dilution (1/20 or 1/30) of SP2 boron nitride destroyed the cell layer only slightly and only under the disc (surface slightly smaller than the disc diameter). On the contrary, dilutions of titanium dioxide particles have shown a low to moderate cytotoxicity.

Accordingly, the SP2 boron nitride particles have surprisingly a lower cytotoxicity as compared to titanium dioxide particles.

### 2. Pre-Maximal Non-Irritant Concentration (Pre-MNIC) determination by topical application

As a first step, the inventors determined the potential skin inflammation resulting from topical administration of SP2 boron nitride solutions.

Two 5 weeks old male albino guinea pigs of Dunkin-Hartley strain were supplied CHARLES RIVER. The animals were housed in group of 5 at the maximum in polycarbonate containers, the flooring of which was covered with dust-free cuttings and the top fitted with a stainless-steel lid with a feeding device and drinking device of 500 ml. The drinking water (tap water from public distribution system) and food were supplied ad libitum. Prior to the test, the animals were kept for a minimum acclimatization period of 5 days, under stabling and nutritional conditions identical to those of the test.

A test composition of 1,3-propanediol comprising 20% (w/w) of SP2 boron nitride was prepared.

This test composition was applied on the dorso-lumbar zone of two guinea pigs shorn beforehand, with occlusive dressing for 24 ± 2 hours, at different concentrations: undiluted (100%), diluted at 75%, 50%, 25%, 10%, 5%, 1%, 0.5%, 0.25% and 0.125% in distilled water.

A macroscopic evaluation of the cutaneous reactions (i.e., (erythema and oedema) was conducted approximately 24 ± 2 hours after removal of the dressing. For this evaluation, all the local or systemic reactions were recorded and graded according to the following grading scale.

The results established that no cutaneous reaction (grade 0) was noted at any tested concentrations of SP2 boron nitride (i.e., 75%, 50%, 25%, 10%, 5%, 1 %, 0.5%, 0.25% and 0.125%).

### 3. Epidermal phototoxicity

Because of this low cytotoxicity and low epidermal inflammation, it was decided to determine the potential epidermal phototoxicity from UV irradiation of SP2 boron nitride solutions.

In fact, and as subjected to UV irradiation in the cornea, it is critical to determine such potential phototoxicity.

This phototoxicity was determined by comparison of the cytotoxicity effect of the test composition on reconstructed Human epidermises, in presence and in absence of exposure to a non-cytotoxic dose of UVA light. The percentage of viability of the treated tissues was calculated with respect to a negative control / vehicle. The difference of viability between the two conditions (exposed and non-exposed) allowed to classify the tested composition as phototoxic or not.

The experiments were done on reconstructed human epidermises (MATTEK EPIDERM) of 0.6 cm² produced in polycarbonate inserts in serum-free and chemically defined medium, featuring normal ultra-structure considered to be functionality equivalent to human tissue *in vivo.*

After the removal of agar used for transportation, the culture medium was renewed using the reagent provided alongside, and the test system was kept in an incubator for at least 1 hour at 37 ± 1 °C, 5 ± 1 % CO2 before use.

A test composition of 1,3-propanediol comprising 20% (w/w) of SP2 boron nitride was prepared. Then, a 1/10 dilution (in PBS) of this composition was prepared. 50 or 25 µl of this dilution was applied topically onto the live tissues and incubated for at least 18-24h with fresh maintenance medium.

At the end of the incubation period, each reconstructed human epidermis was transferred to another 24-wells plate containing 300 µL/well of HBSS. Half of the reconstructed human epidermises were kept protected from light in a dark box as UV-A non-exposed reconstructed human epidermises. The other half of the reconstructed human epidermis were irradiated by UV-A at RT with 6 J/cm² in the presence of the test composition.

After irradiation, all epidermises (irradiated or not) were rinsed with PBS and post-incubated for 18 to 24 hours in fresh medium at 37 ± 1 °C, 5 ± 1 % CO2. Then, cell viability for each reconstructed human epidermis was assessed using the MTT assay. The results are presented in the following table.

| Solution | Relative viability in non-Irradiated plate (% as compared to negative control) | Relative viability in Irradiated plate (% as compared to negative control) | Viability decrease (fold between Non-Irradiated and Irradiated plates) |
|---|---|---|---|
| Negative control (PBS) | 100.00 | 100.0 | - |
| Positive control 1 (Chlorpromazine at 0.3 mg/ml in PBS) | 65.7 | 6.7 | 9.8 |
| SP2 Boron Nitride 10% solution | 75.2 | 51.8 | 1.4 |

Finally, the results established that a composition of SP2 Boron Nitride was predicted to be non-phototoxic according to the applicable prediction model.

### 4. Ocular toxicity

The potential intrinsic property of the composition to produce specific ocular irritation was assessed in healthy, young adult albino rabbits of female sex with a body weight not less than 2 kg were used. The animals were nulliparous and non-pregnant. The animals were kept in their cages (single-caged) for at least 5 days prior to test start to allow for acclimatization to the laboratory conditions. Both eyes of each experimental animal were examined for evidence of ocular abnormality within 24 hours before test start.

A test composition of 1,3-propanediol comprising 7% (w/w) of SP2 boron nitride was prepared. A volume of 0.1 ml of this test composition was instilled initially into the lower conjunctiva! sac of the right eye of three rabbits. The eyelids were kept shut for 1 s following instillation. The left eye of each animal served as a control and was treated with physiological saline solution.

The eyes were examined for evidence of ocular abnormality by means of a slit lamp (1±0.1) hour, (24±2), (48±2) and (72±2) hours after test substance application.

The reactions were analyzed according to DIN EN ISO 10993-23:2021-10 by comparing exposure to control eye.

The results established surprisingly that no eye abnormalities were detected in all test animals, within the observation period of 72 h. Accordingly, the composition comprising boron nitride particles meets the requirements of DIN EN ISO 10993-23:2021-10 and is considered non eye irritating.

### 5. keratopigmentation potential

Because of the established cellular and animal non-toxicity of SP2 boron nitride compositions, notably for ocular, it was decided to determine the potential of boron nitride solutions for keratopigmentation.

Dye solutions corresponding to compositions comprising different pigments mixtures up to 20% (w/w), with titanium dioxide particles or boron nitride particles (SP boron nitride), were prepared and gamma irradiated. The detail of each composition depended on the final expected color.

Before keratopigmentation, all patients were subjected to eyes local anesthesia using anaesthetic eye drops.

Then, a micro-tunnel was created in each patient's cornea (not in the iris) without touching any internal tissue using a femtosecond laser.

In the minutes following micro-tunnels creation, about 0.5 mL of the dye solution was injected in each of these two micro-tunnels using a 3 mL syringe and a cannula.

Half of the patients were injected using dye solutions with titanium dioxide particles.

The rest of the patients were injected using dye solutions with boron nitride particles.

Following the intervention, the patients wear sunglasses for a few days.

The pigment adherence/ spreadability - i.e., pigment short term stability following injection in the cornea- was determined in the next days, and the pigment non-fading characteristic -i.e., pigment long-term stability in the cornea - in the next months.

The obtained results are presented in the following table.

| Color | Dye solution with titanium dioxide particles | | Dye solution with boron nitride particles | |
|---|---|---|---|---|
| | Pigment adherenc e | Pigment non-fading characteristic | Pigment adherence | Pigment non-fading characteristic |
| Blue | + | + | ++ | ++ |
| Light blue | + | Getting white | ++ | ++ |
| Green | ++ | ++ | ++ | ++ |
| Light green | ++ | + | n.d. | n.d. |
| Clear brown | + | Getting yellow over time | ++ | ++ |
| brown | ++ | ++ | +++ | n.d. |
| Chocolate | ++ | ++ | n.d. | n.d. |

| | | | | |
|---|---|---|---|---|
| n.d. not determined | | | | |

The results established that the use of boron nitride particles provide instead of titanium dioxide particles provides a better adherence to the pigment mixtures.

Moreover, the results further established that the use of boron nitride particles provide instead of titanium dioxide particles provides a better color stability (i.e., non-fading characteristics) to the pigment mixtures over time.

## Claims

1. A composition for keratopigmentation comprising pigments, **characterized in that** said pigments comprise boron nitride particles.

2. The composition of claim 1, wherein said pigments do not comprise any titanium dioxide particles.

3. The composition of any one of claim 1 or 2, wherein the boron nitride particles are selected in the Standard Platelets grades (SP) group comprising SP1, SP2, SP5, SP6 or SP8 boron nitride, and preferably boron nitride particles are SP2 boron nitride.

4. The composition of any one of claims 1 to 3, wherein the boron nitride particles represent from 0.1 to 20% (w/w) of the composition, preferably from 0.5 to 15, and most preferably from 1.0 to 10% (w/w) of the composition.

5. The composition of any one of claims 1 to 4, wherein the pigments represent from 1.0 to 30% (w/w) of the composition, preferably from 1.5 to 25%, typically from 2.0 to 20%, and most preferably from 2.5 to 15% (w/w) of the composition.

6. The composition of any one of claims 1 to 5, wherein the pigments are selected in the group comprising Black 6/7 (CI 77266), Black 11 (CI 77499), Blue 29 (CI 77007), Green 17 (CI 77288), Green 18 (CI 77289), Manganese violet (CI 77742), Red 101 (CI 77491), violet 27 (CI 77510), Yellow 42 (CI 77492), Yellow 119 (CI 77496), and any combination thereof.

7. The composition of any one of claims 1 to 6, wherein the composition further comprises a pigments' carrier selected in the group comprising water, glycerin, propane-1,2-diol, Propane-1,3-diol, and combination thereof.

8. The composition of claim 7, wherein the carrier represents from 60 to 99% (w/w) of the composition, preferably from 80 to 98% (w/w), and most preferably from 85 to 97% (w/w) of the composition.

9. The composition of any one of claims 1 to 8, wherein the composition further comprises one or more additive such as dispersants, humectants, or pH regulators.

10. The composition of claim 9, wherein the additives represent less than 15% (w/w) of the composition, preferably less than 10% (w/w), and most preferably less than 5% (w/w) of the composition.

11. A composition as defined in any one of claims 1 to 10, as a pharmaceutical.

12. A composition as defined in any one of claims 1 to 11, for treating an iris defect a subject by keratopigmentation.

13. The composition for treating an iris defect in a subject by keratopigmentation of claim 12, wherein the iris defect is select in the group comprising corneal opacities, corneal scars, aniridias, colobomas, heterochromias, and iris lacks pigmentation.

14. The composition for treating a subject by keratopigmentation of claim 12, wherein the keratopigmentation is femtosecond laser-assisted intrastromal keratopigmentation (FLAK).
